# EUROPEAN PATENT SPECIFICATION

(11) **EP 4 051 332 B1**
(45) Date of publication and mention of the grant of the patent: **18.06.2025**
(21) Application number: 20825156.1
(22) Date of filing: 02.11.2020
(51) Int. Cl.: A61M 1/06

(54) **IN-BRA PASSIVE SUCTION-BASED MILK COLLECTION DEVICE**
AUF PASSIVEM SAUGEN BASIERTE IN BH TRAGBARE MILCHSAMMLUNGSVORRICHTUNG
DISPOSITIF PORTABLE DANS LA BRASSIÈRE POUR COLLECTER DU LAIT PAR SUCCION PASSIVE

(30) Priority: 31.10.2019 GB 201915805; 13.02.2020 GB 202001955; 07.10.2020 GB 202015919
(43) Date of publication of application: 07.09.2022
(73) Proprietor: Chiaro Technology Limited, London EC1N 8LE (GB)
(72) Inventor: O'TOOLE, Jonathan, London EC1N 8LE (GB); CARR, Andrew, London EC1N 8LE (GB); SARDAR, Nick, London EC1N 8LE (GB); ROSS, Clare, London EC1N 8LE (GB)
(74) Representative: Collins, Emily Jane
(86) International application number: PCT/GB2020/052775
(87) International publication number: WO 2021/084283

(56) References cited:
- US-A1- 2008 262 420
- US-A1- 2014 378 946
- US-B1- 6 440 100

## Description

### BACKGROUND OF THE INVENTION

### 1. Field of the Invention

The field of the invention relates to an in-bra passive suction-based collection device for collecting breast milk.

### 2. Description of the prior art.

Semi-active breast pumps, such as silicone breast pumps, are an emerging category within the growing breast pump market. Semi-active breast pumps are designed to collect let down milk when breastfeeding/pumping on the other breast.

Silicone breast pumps encourage increased levels of breastmilk let down through the application of a constant vacuum applied to the nipple. They often include a compressible milk pouch that is squeezed prior to being placed onto a user's breast; the milk pouch also acts as a collection vessel. When placed onto the breast and correctly aligned and sealed around the nipple, a vacuum is created; the negative pressure draws out milk from the nipple that is then collected inside the milk pouch.

As milk flows into the collection vessel, the vacuum level decreases until milk ceases to flow. If enough milk is available, vacuum pressure will decrease until ambient pressure is reached, where it is possible for the silicone breast pump to fall off the exposed breast.

If the user wishes to collect a greater volume of milk, the user must remove and re-apply the silicone breast pump, to ensure negative pressure is repeatedly applied up to the maximum collection capacity of the device.

When milk doesn't flow, or stops flowing, the seal of the breast pump around the nipple needs to be broken to release the vacuum and restart the suction. This is done by first removing the breast pump and then re-attaching it onto the breast.

Current offerings are often restrictive, in that they cannot be worn behind clothing without risking falling from the user and causing mess and/or milk wastage; they restrict the user from moving during use, and inhibit peace of mind in case the product needs sudden attention to avoid milk spillage. They can be uncomfortable, in that if the user sets a vacuum level that is not comfortable, the resetting of vacuum is cumbersome enough that they may choose to continue in discomfort instead of finding a comfortable vacuum level to continue at. Ultimately they do not match parents' modern lifestyles.

The present invention addresses the above vulnerabilities and also other problems not described above.

US 2008/262420 discloses a human breast milk collection device that fits into a mother's existing nursing or standard brassiere or comprises a nursing brassiere and milk collection system. The device can be attached to a regular electric pump or manual pump for active milk collection and also can be used without a pump for passive milk collection. A breast adaptor is a funnel-shaped inlet coupled to a reservoir, wherein when the breast is inserted into the breast adaptor, the expressed breast milk is expressed into the reservoir and is stored there until the device is removed and emptied.

US 6,440,100 B1 discloses a concealed apparatus for hands free breast milk pumping and storage includes one or more low profile nipple caps held in place beneath a support brassiere. The system permits concealed, hands free breast pumping in a public environment without any remarkable change in the user's visible appearance. Milk is expressed from the breasts through low profile nipple caps linked to a reservoir via a milk storage conduit. A vacuum is applied to the storage reservoir by any suitable means such as an electric or manual vacuum pump. Suction is thus conveyed through related system components to express milk from the breasts and draw it into the storage reservoir.

### SUMMARY OF THE INVENTION

Wearable passive suction-based milk collection device (1), the device comprising a compressible milk pouch (10) with a nipple hole or opening (20) in a rear face of the compressible milk pouch, and in which the rear face of the compressible milk pouch is configured to seal against at least part of the user's breast when the pouch is compressed by a user in order to create a negative air pressure inside the pouch, providing suction onto the nipple thereby drawing milk from the nipple for collection in the milk pouch; and in which the device is shaped, at least in part, to fit inside a bra, in which the compressible milk pouch includes or is connected to an air valve (12) to enable the user to reduce the level of negative pressure inside the milk pouch whilst the device is being worn.

### BRIEF DESCRIPTION OF THE FIGURES

Aspects of the invention will now be described, by way of example(s), with reference to the following Figures, which each show features of various implementations of the invention including optional features that may be utilised:
- **Figure 1**: is a perspective view of a passive suction-based collection device.
- **Figure 2**: is a back view of a passive suction-based collection device.
- **Figure 3**: is the frame and compressible milk pouch shown side by side and assembled.
- **Figure 4**: is a perspective view of a frame.
- **Figure 5**: is a perspective view of a rendered drawing of the passive suction-based collection device.
- **Figure 6**: is a perspective view of a rendered drawing of the passive suction-based collection device.
- **Figure 7**: is showing pictures of the entire device resting on its front and upright on its base.
- **Figure 8**: is another example of a passive suction-based collection device comprising an optional, or in-built, sealing solution.
- **Figure 9**: is a passive suction-based collection device comprising an optional protection plate.
- **Figure 10**: is a passive suction-based collection device comprising an optional NRV sealing clip.
- **Figure 11**: is illustrating the steps of pouring liquid out of the wearable device.
- **Figure 12**: shows pictures illustrating examples of squeezing or priming the device.
- **Figure 13**: shows a table summarizing the key specifications.
- **Figure 14**: shows the manufacturing steps in moulding the pouch.
- **Figure 15**: shows the manufacturing steps in moulding the pouch.
- **Figure 16**: shows an assembled compressible pouch comprising multi-parts.
- **Figure 17**: shows a disassembled compressible pouch comprising multi-parts.
- **Figure 18**: shows an assembled compressible pouch comprising multi-parts.
- **Figure 19**: shows a disassembled compressible pouch comprising multi-parts.
- **Figure 20**: shows a compressible pouch comprising multi-parts.
- **Figure 21**: shows a compressible pouch comprising multi-parts.
- **Figure 22**: shows a compressible pouch comprising multi-parts.
- **Figure 23**: shows an optional sealing feature configured to seal the nipple hole on the rear surface of the pouch.
- **Figure 24**: is a passive suction-based milk collection device with the compressible pouch and frame are integrated as a single part.
- **Figure 25**: shows examples of different frame structures.
- **Figure 26**: is the passive suction-based collection device connected to an external storage container.
- **Figure 27**: is a compressible pouch including a valve mechanism and a toggle feature.
- **Figure 28**: is a passive suction-based milk collection device is further configured to enable a user to increase the suction provided by the device.
- **Figure 29**: is a compressible pouch including an insertable external rigid protection.
- **Figure 30**: is a carrying case.

### DETAILED DESCRIPTION

An implementation of the invention, is a passive suction-based collection device that is shaped, in use, at least in part, to fit inside a bra.

When breastfeeding or pumping on just one breast, let down can occur from the other breast, as a reflex. The passive suction-based collection device can be placed on the other breast to collect this 'bonus milk' and can also be used as a standalone pumping device.

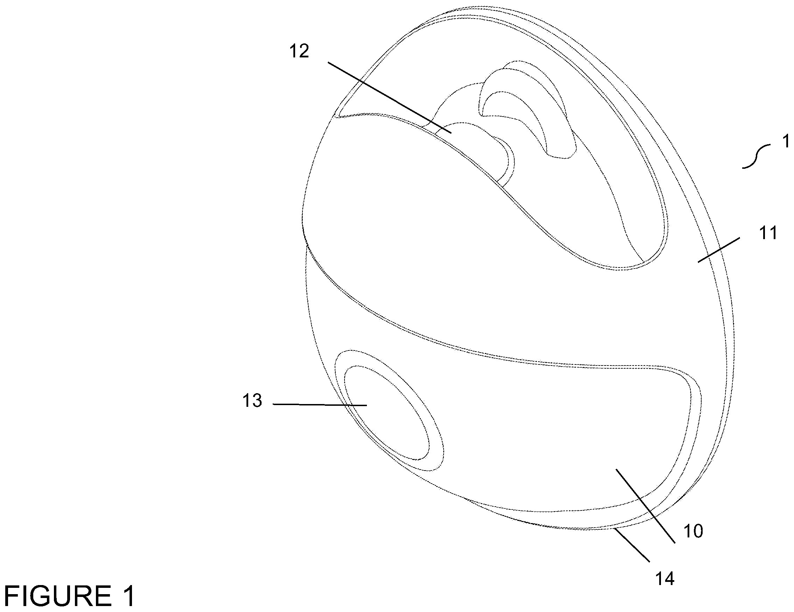

With reference to Figure 1, a perspective view of the passive suction-based collection device is shown. The passive suction-based collection device or wearable device is used for extracting and collecting breast milk by encouraging increased levels of breastmilk let down through the application of a constant vacuum to the nipple. The device has a capacity of collecting approximately up to 4 US fluid ounces (120ml).

Alternatively the device may also be configured to have a capacity of about 5 US fluid ounces (148ml).

The wearable device combines a discreet, ergonomic design with semi-active suction for the efficient initiation of let down and collection of 'bonus milk', all without restricting the user's movement. An additional advantage of the device when it is worn in-bra is that it is protected from being grabbed, knocked or kicked by a baby during breastfeeding.

The device comprises a compressible part or milk pouch 10 and a frame 11 that is configured to fit over the compressible milk pouch.

The front surface of the compressible milk pouch includes a flat portion 13 so that the entire device can rest stably on its front on a surface; while preventing any milk spillage. The frame or exoskeleton also includes a flat base 14 so that the entire device can rest upright on a surface.

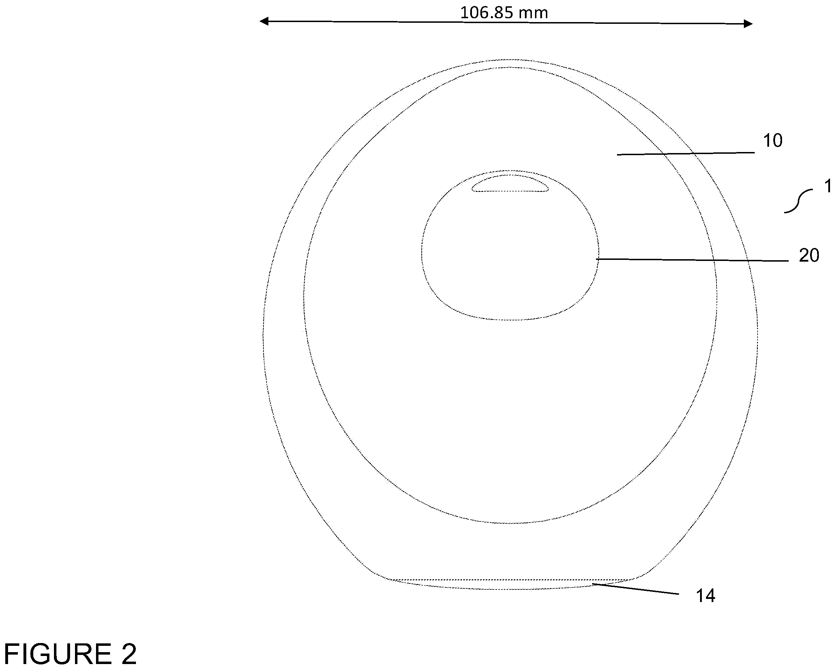

With reference to Figure 2, a back view of the wearable device is shown. The rear surface of the compressible milk pouch 10 is shaped to fit against a breast and comprises an opening or hole 20. When placed onto the breast, the rear surface of the compressible milk pouch forms a seal or at least a partial seal with the breast around the opening 20. This in turns creates a vacuum inside the compressible milk pouch and simulates suction of the nipple to extract breast milk and collect it inside the compressible milk pouch.

In standard solutions, squeezing the milk pouch leads to the pouch being twisted out of shape, which in turns reduces nipple clearance and makes a correct nipple alignment difficult to achieve.

In comparison, the device is configured such that any warping of the area of the pouch around the nipple, when the device is squeezed prior to being placed on the breast, is limited. This is due, in particular, to the shape of the pouch as well as the location and shape of the squeezable portion of the pouch as compared to the shape and location of the area around the nipple.

Further, the ability for the device to be worn in bra, means that the device can be positioned before any squeezing or priming has taken place, giving the user the convenience of positioning the device in its rest shape. Therefore, a correct nipple alignment when placing the device is easier to achieve.

The compressible milk pouch includes a Non-Return Valve, or 'NRV' 12 (as shown in Figure 1). The NRV allows air to exit the device whilst the device is being primed or squeezed and hence sealed to the breast.

Allowing air to flow out from the milk pouch via the NRV when the device is being primed or squeezed advantageously reduces air from being substantially evacuated through the nipple opening. Hence, the device can be easily primed comfortably in situ. Discretion is also improved by the reduction in the likelihood of evacuation of air through the nipple opening creating audible noise, which may draw attention to the user.

The NRV allows a user to adjust the level of negative pressure inside the chamber, such as for comfort or pumping efficiency reasons (personal choice and varies from person to person).

Hence a controlled reduction of the vacuum inside the milk pouch is achievable via a simple interaction of the user with the wearable device, such as a light finger touch or squeeze of the NRV that opens a very small cut on the valve to let some air back into the housing and reduce the vacuum level.

Therefore, the level of the vacuum inside the milk pouch is easily adjustable by the user whilst the device is positioned and sealed onto the user's breast. Further, adjusting the vacuum inside the milk pouch does not impact milk flow since the air valve can be opened for a moment whilst the device is being used and milk is flowing to reduce the internal air pressure. This is because the valve is located, and can be interacted with, independently of the pouch sealing to the nipple and drawing a vacuum around it. Therefore the primary function of the device can continue, uninterrupted, whilst the user chooses to adjust the vacuum level.

Also, if the air valve is a two way air-valve, then the milk pouch can be squeezed, whilst the device is being worn, to increase the internal vacuum; this can prime the device to start collecting milk, or start a subsequent session to continue milk collection. As an example, a user may typically re-squeeze the milk pouch every 3 minutes, and do this three times for a total pumping session of 12 minutes. Milk will flow largely continuously throughout this 12 minute session. However, the amount a user interacts with the device and/or length of a session, is a personal choice and varies from person to person.

The NRV also allows the user to squeeze air out of the compressible milk pouch when it is being body worn and is underneath clothing, for example, to increase the suction pressure - e.g. the user can readily squeeze the housing though their clothing. In comparison, the user needs to break the seal around the nipple to achieve the same effect using other standard silicone breast pumps, making it impossible or at least difficult to do so underneath clothing.

Further, activating the NRV with a finger push allows the user to lower the vacuum inside the pouch, or release the vacuum totally, for easier and more comfortable removal from the breast.

The compressible milk pouch and the NRV can be a single, integral one-piece moulded item (as shown).

Alternatively, the NRV may also be removable or separate from the compressible milk pouch 10.

Alternatively, the NRV may also be integrated with the frame itself. In this case, assembling the frame onto the pouch would also attach the NRV to the pouch.

Examples of dimensions are also provided in Figures 1 and 2.

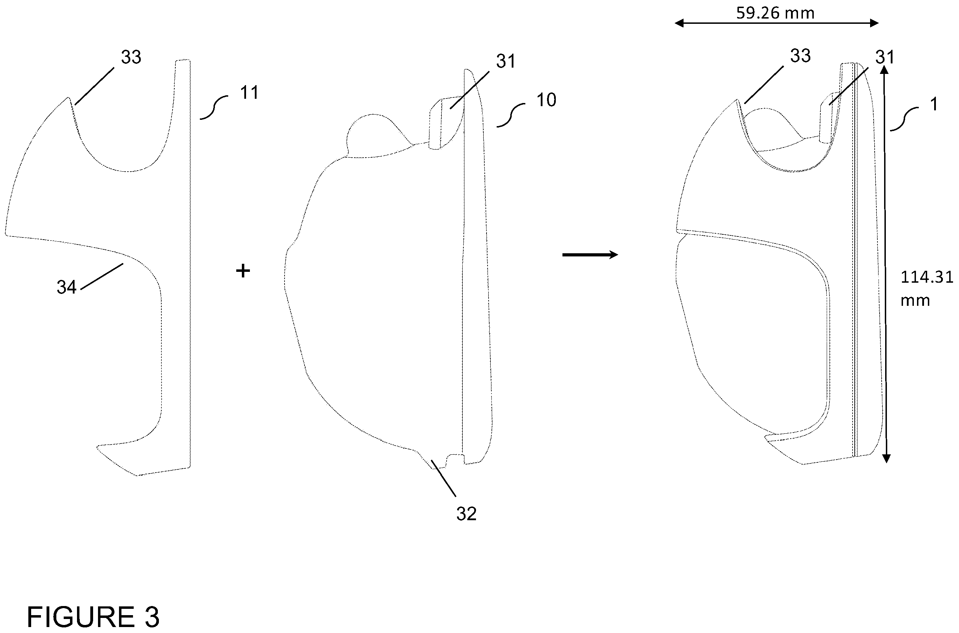

With reference to Figure 3, the frame 11 and compressible milk pouch 10 are shown side by side and assembled 1.

The frame 10 substantially fits over the compressible milk pouch and is retained in attachment with the compressible milk pouch. Attachment features located on the compressible milk pouch and frame ensure the two parts are fixed securely, in use. The compressible milk pouch includes a latch feature 31 located at its top and a bump 32 located at its base.

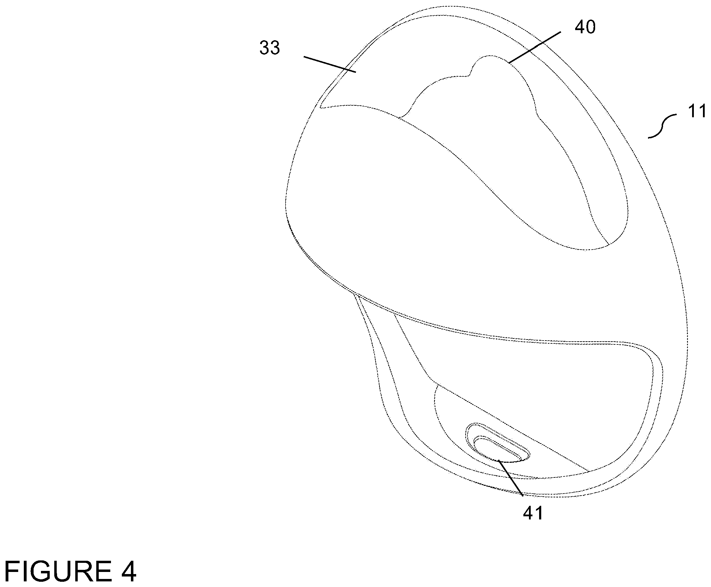

With reference to Figure 4, the frame includes a feature 40 to secure the latch 31 at the top and an opening 41 at its base to receive the compressible milk pouch's bump 32. Tactile and/or audible feedbacks confirm the frame 11 and compressible milk pouch 10 are properly assembled for normal use.

Additionally, the frame 11 includes an opening or window 33 on its front surface to provide, in use, a view of a portion of the breast and of the nipple for easy nipple alignment and to enable an easy access of the NRV to a user. Another opening or window 34 on the front surface of the frame enables easy access of the compressible milk pouch to the user. The opening 34 is shaped so that the compressible milk pouch can be easily squeezed using one hand.

The removable frame 11 provides a number of key features, such as, but not limited to:
- allows the compressible milk pouch 10 to expand (as it fills with milk) when the device is worn inside a bra. The bra could otherwise limit the ability for the compressible milk pouch to expand.
- provides a rest shape for the compressible milk pouch.
- protects the compressible milk pouch from being pushed towards the nipple, such as from being crushed by a bra or any other clothing.
- provides greater nipple clearance as compared to other silicone breast pumps when worn in a bra.
- helps prevent the device from being accidentally compressed when in storage and containing milk, preventing spilling and wasting of valuable expressed milk.
- helps protect the non-return valve from accidental activation, such as from pressing the bra.
- allows a higher vacuum level inside the milk pouch, as compared to without using a frame.

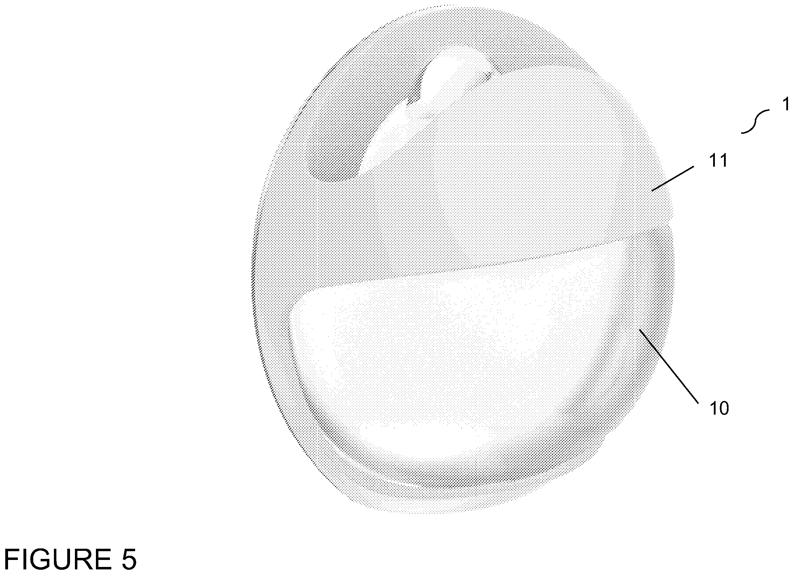

With reference to Figure 5, a perspective view of a rendering of the passive suction-based collection device is shown. The compressible milk pouch is substantially transparent or optically clear to give an unobstructed view of the breast or nipple area. The compressible milk pouch is made of an elastic material. The frame is opaque and made of hard plastic. The materials are chosen to provide ease of use and cleaning.

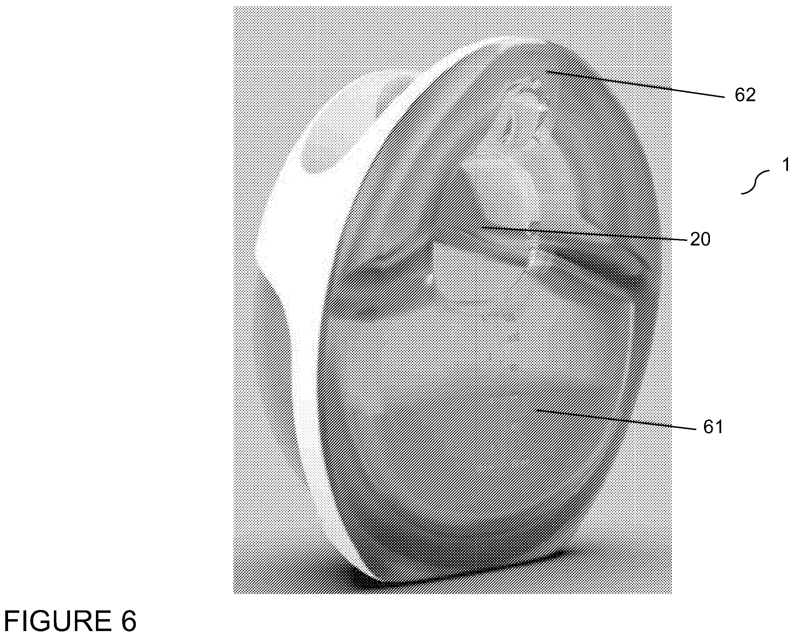

With reference to Figure 6, a rear view of a rendering of the passive suction-based collection device is shown. The rear surface of the compressible milk pouch includes fill lines providing an indication of how much the compressing milk pouch has been filled with milk. As shown, the fill lines may be directly read when the device is in its upright position.

The rear surface of the milk pouch also provides a milk pouring section or spout 62 near the nipple hole 20 providing an indication on where and how to pour collected milk out of the device.

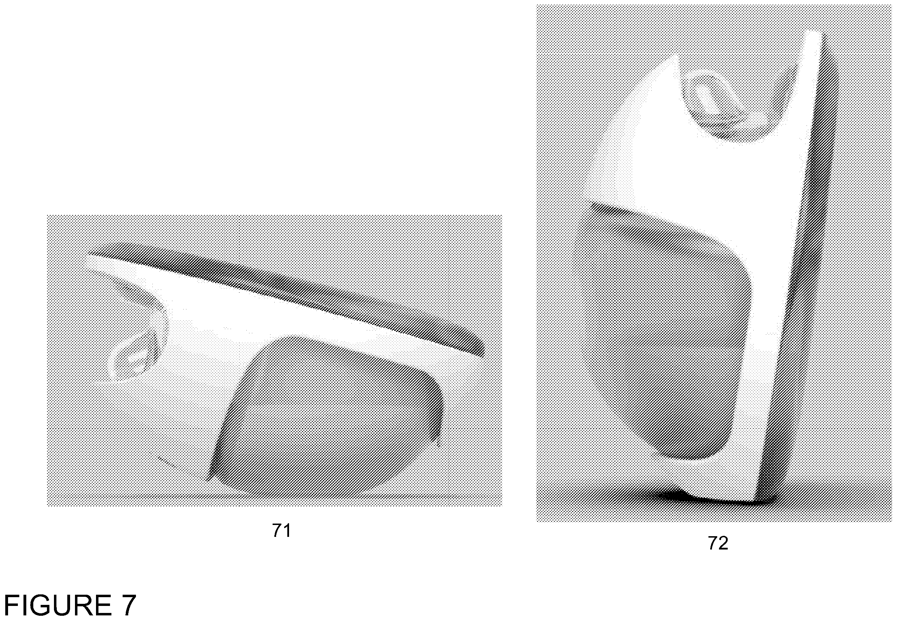

With reference to Figure 7, renderings are shown of the entire device resting stably on its front 71 and upright on its base 72 on a flat surface.

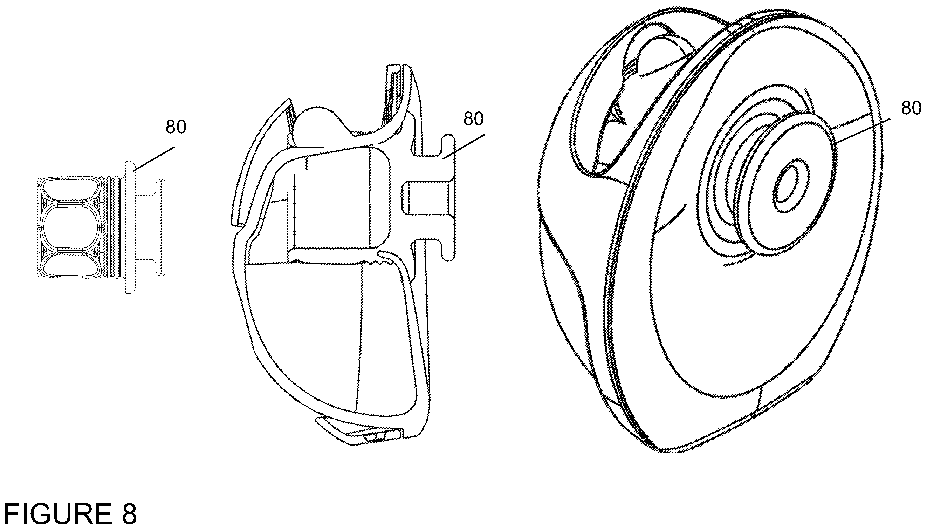

With reference to Figure 8, an optional, or in-built, sealing device 80, such as a stopper, may also plug the nipple opening, preventing leaks, thus turning the device into a milk storage device. Sealing or plugging the nipple opening, also keeps the nipple-contact areas clean when the device is in storage. The sealing device may also be attached to the wearable device, to help prevent losing it.

Additionally, the sealing device may also be configured to seal the NRV in order to prevent any leaks from the NRV. The sealing device may either seal the NRV from an internal area directly behind the NRV or from an outside area of the device.

As shown in Figure 8, the silicone stopper 80 may also be configured to seal both the nipple hole and the NRV from the inside of the device, as shown in the cross section. The rotationally symmetrical design enables both the nipple hole and the NRV to be effectively sealed, regardless of the rotational orientation of the stopper.

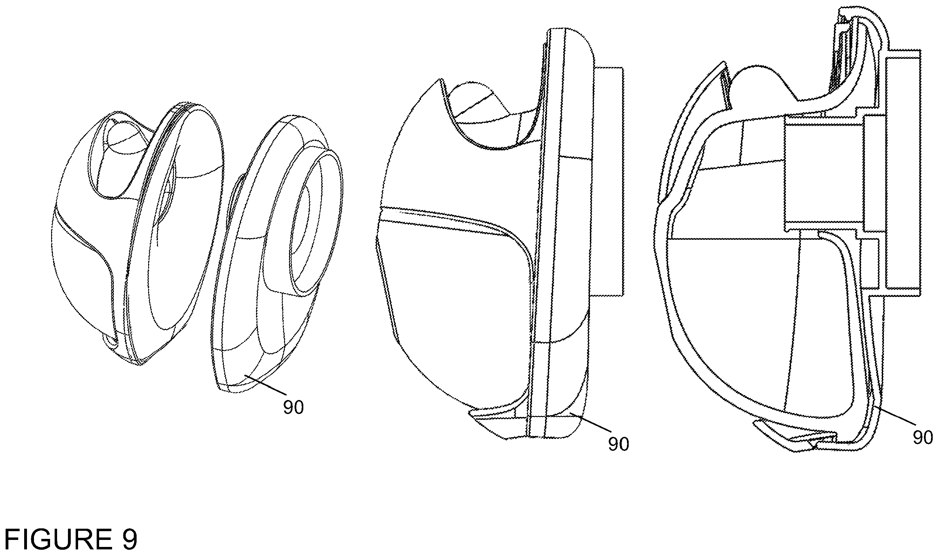

With reference to Figure 9, an optional protection plate 90 is shown that provides a combined stopper and keep-clean protection plate. The protection plate may be pushed into the rear surface of the pouch, when not in use, in order to keep any surfaces in contact with the breast clean such as before or after use, during transport or in milk storage configuration.

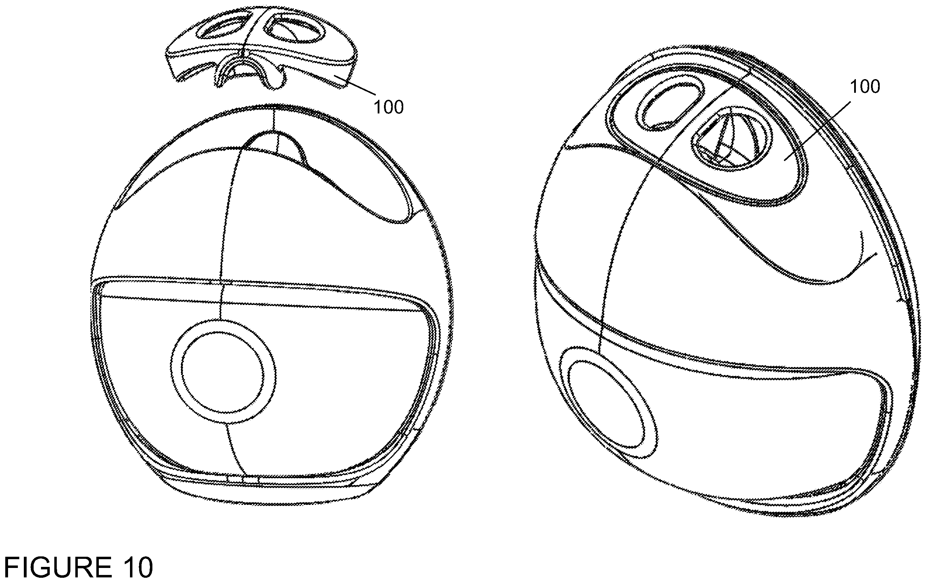

With reference to Figure 10, an external NRV sealing clip 100 is provided. The sealing clip engages with the NRV, making it resistant to opening, even when the pouch is pressurized due to squeezing.

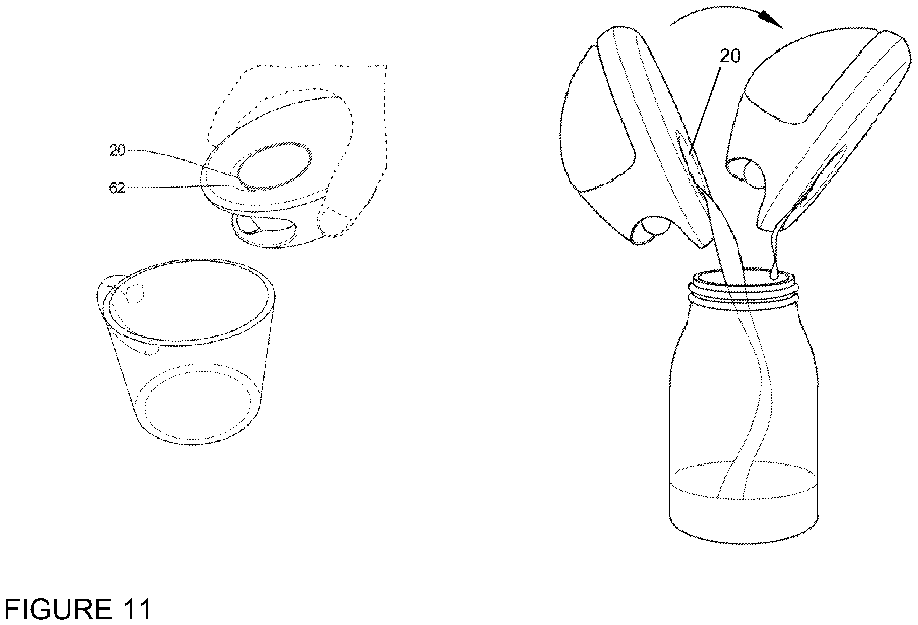

With reference to Figure 11, pictures showing the steps of pouring liquid out of the wearable device are shown. Collected milk can easily be poured out of the device from the opening 20. In addition, a milk pouring section 62 (or spout) is located near the nipple opening. Visual indicator, such as specific texture on the compressible milk pouch provides an indication on where and how to pour the collected milk out of the device.

Example of measurements are now provided:
- Maximum vacuum achievable is in the order of 100mmHg, achieved by expelling almost all air.
- General working vacuum is in the order of 35-45mmHg.

Milk collected may be dependent on one or more of the following: 1) availability of milk from a mother, 2) efficiency of the first squeeze to the compressible milk pouch, to expel air through the NRV, 3) number of 're-squeezes'.

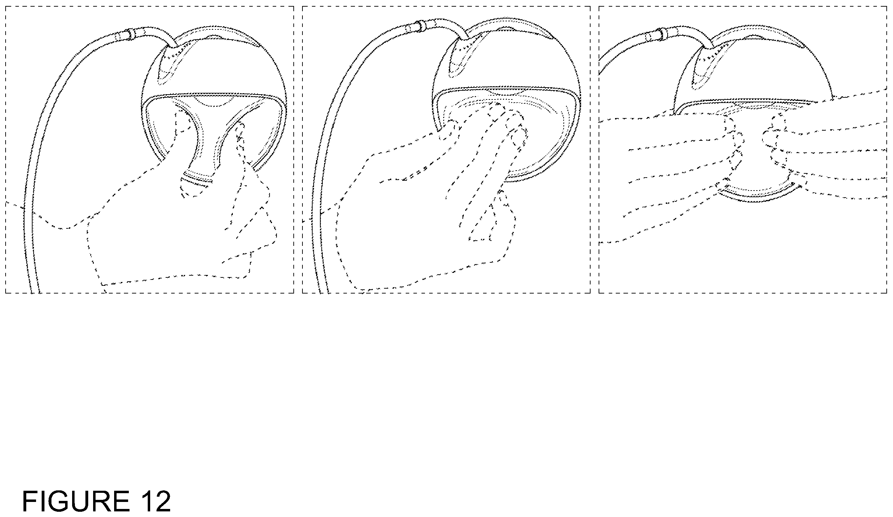

Figure 12 shows pictures illustrating examples of squeezing or priming the device. An example illustrating an equivalent of a squeeze vs. the volume of air expelled and amount of milk collected is now provided:
- If 1 squeeze = 30ml air, then we could expect 120ml milk from 4x re-squeezes if milk flow is good.
- Re-squeezes are typically every 3 minutes, so the average productive period is about 12 minutes. However, mums are seen to wear the device for up to 30 minutes as a wearable collection device.
- Squeeze, or re-squeeze may also vary depending on a number of factors such as the time of day or last feeding time.

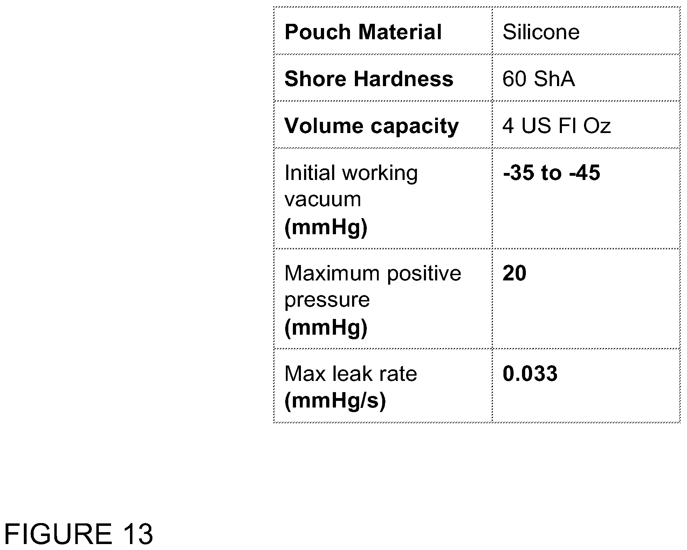

Figure 13 shows a table summarizing the key specifications of the device.

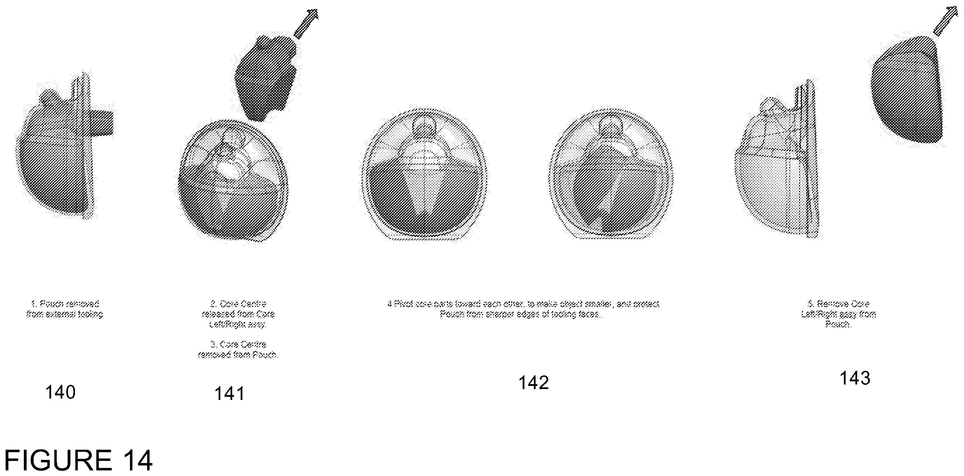

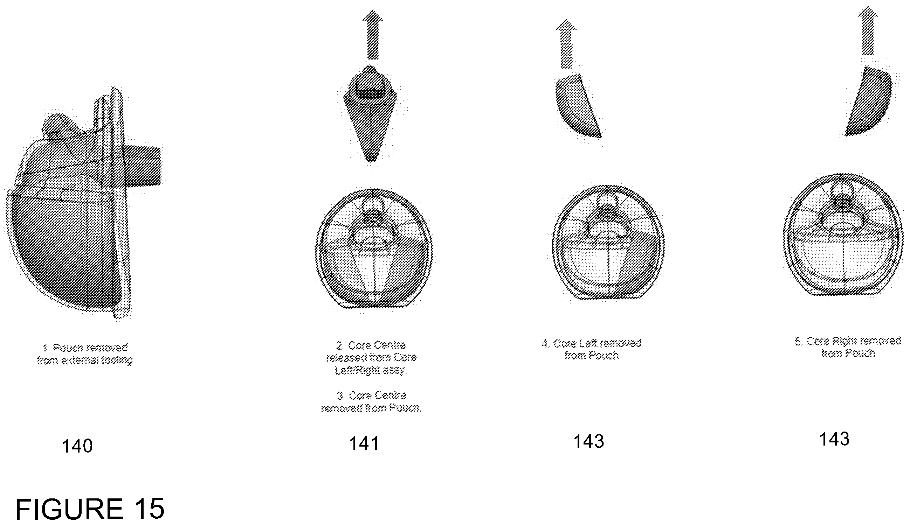

With reference to Figures 14 and 15, the manufacturing steps in moulding the pouch are illustrated. Moulding large internal cavities for silicone breast pumps is often challenging. As shown, the moulding of the compressible pouch is achieved using a multi-part core removal, in which the core is segmented to allow easier removal of the core via multiple manufacturing steps.

The steps are as follows:
· the pouch is removed from an external tooling 140.
· a centre core is released from a left/right core assembly 141.
· the centre core is removed from the pouch 141.
· the remaining parts of the core are pivoted towards each other, to make them appear smaller and to protect the pouch from sharper edges of tooling faces 142.
· the left core is removed from the pouch 143.
· the right core is removed from the pouch 143.

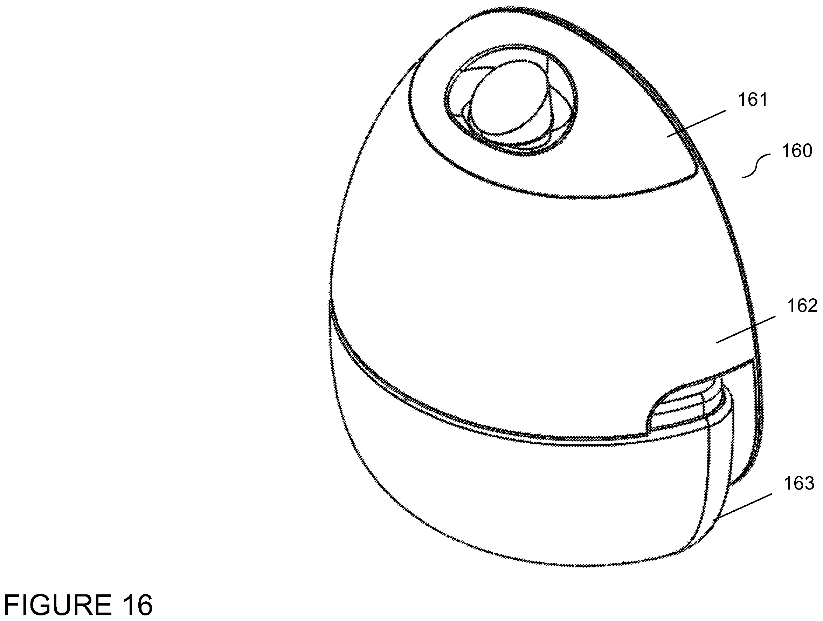

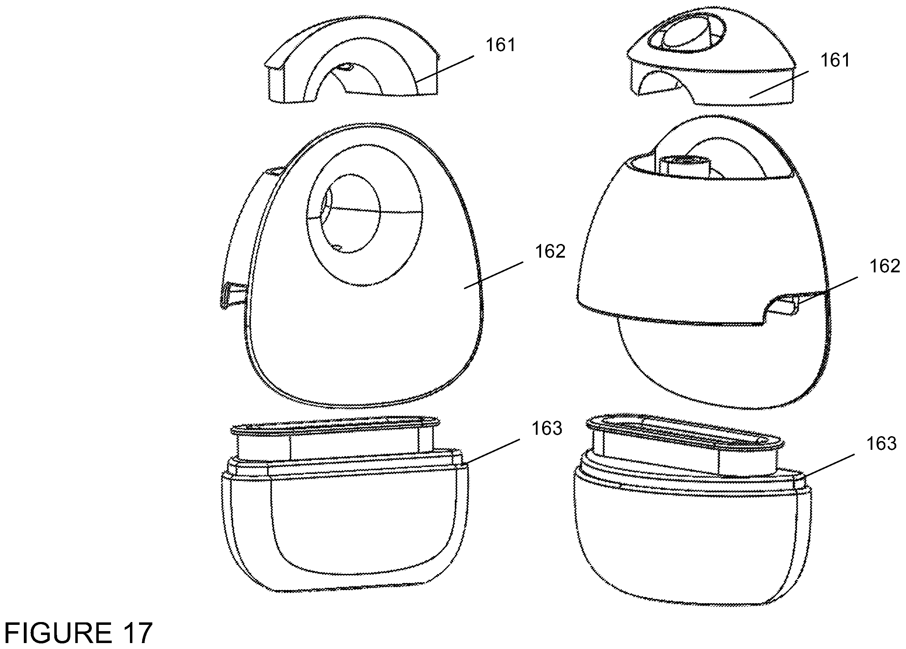

With reference to Figures 16 and 17, a compressible pouch comprising multi-parts is shown assembled 160 and disassembled. Providing a multi-part pouch enables the use of a removable breast-shield component 162, which is then interchangeable in order to provide different nipple size options. The squeezable element 162 is provided with a large opening in order to aid in manufacturing.

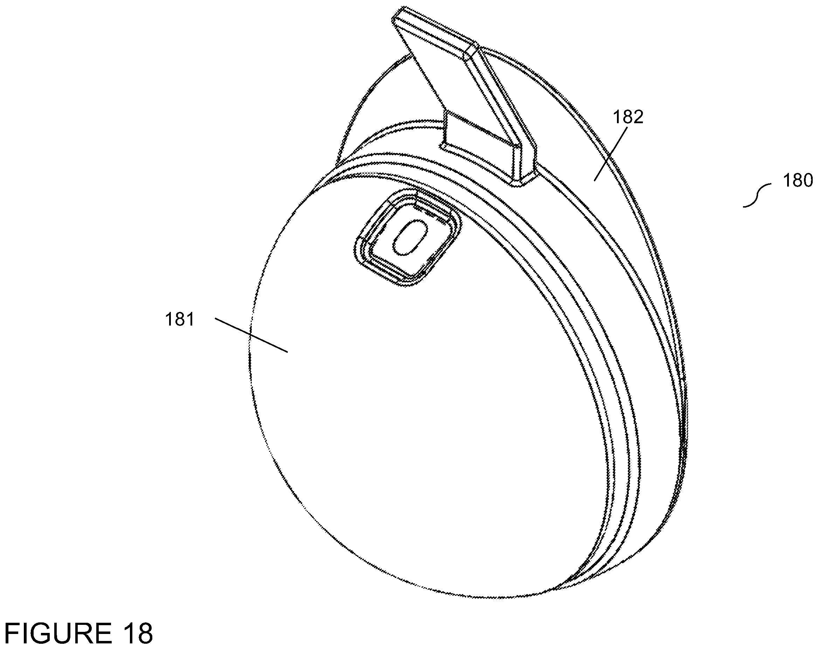

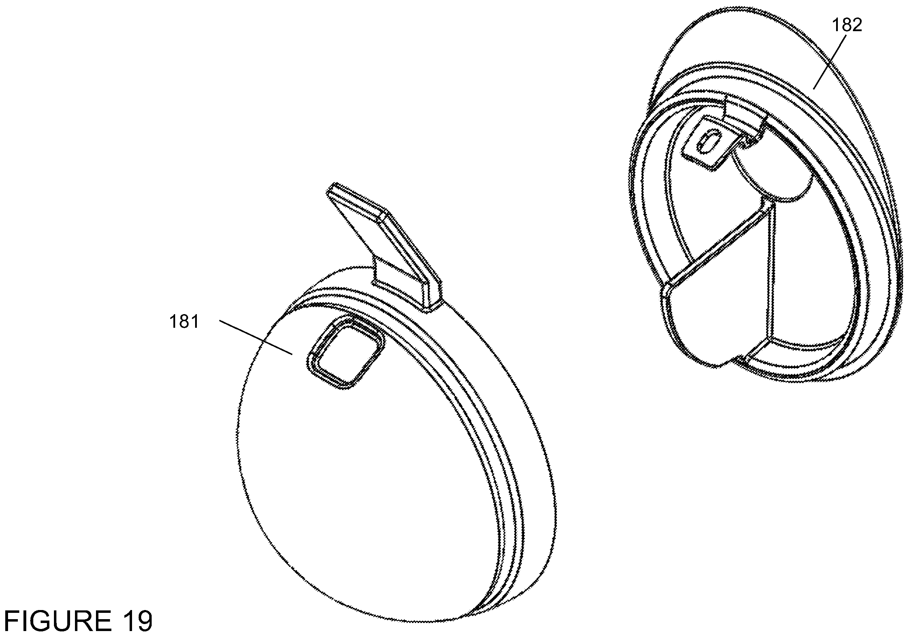

With reference to Figures 18 and 19, a compressible pouch comprising two removable parts is shown assembled 180 and disassembled. An elastomer squeezable part 181 connects to a rigid part 182. The rigid part includes a nipple hole or opening to receive a nipple. The two parts may be connected using snap-fit sealing features. The flexible part provides a large chamber for collecting milk. In addition, the large opening of the flexible part enables easier manufacturing, cleaning and drying.

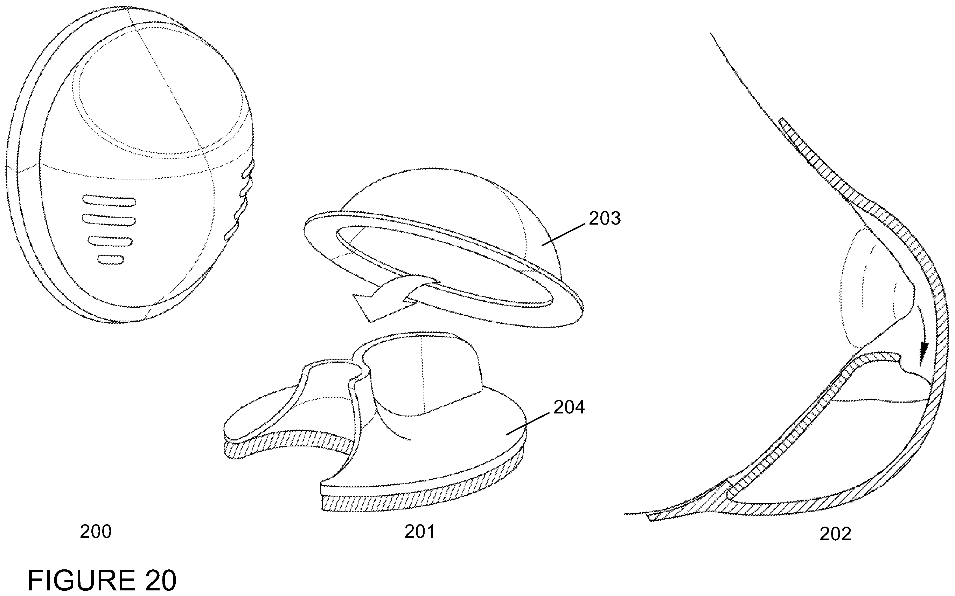

With reference to Figure 20, a compressible pouch comprising two removable parts is shown, assembled 200, disassembled 201 and in use 202. The compressible pouch is configured to be easily manufactured and allows easy disassembly for cleaning. A silicone pouch 203 is configured to snap around a hard inner moulding 204 including a central spine. In use, when the passive suction-based milk collection device is worn by a user, the opening of the hard moulding is configured to create a pathway such that any milk extracted from the nipple is collected in the pouch below the nipple-line.

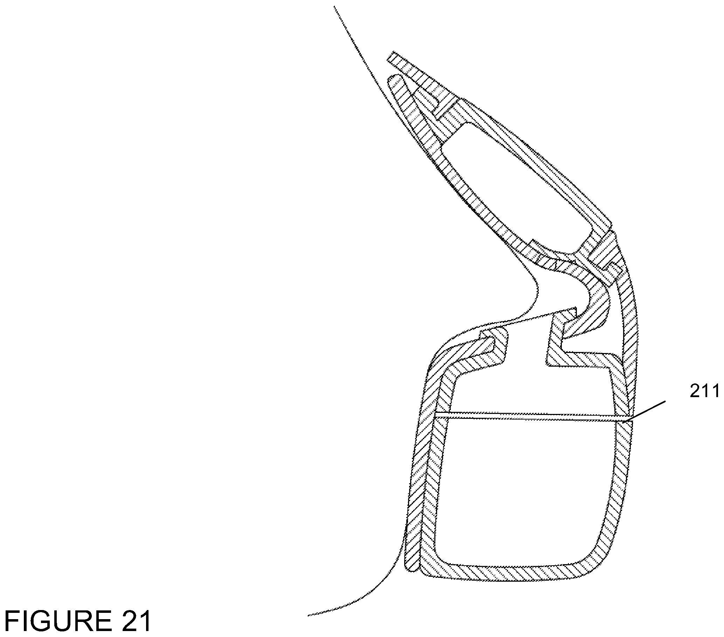

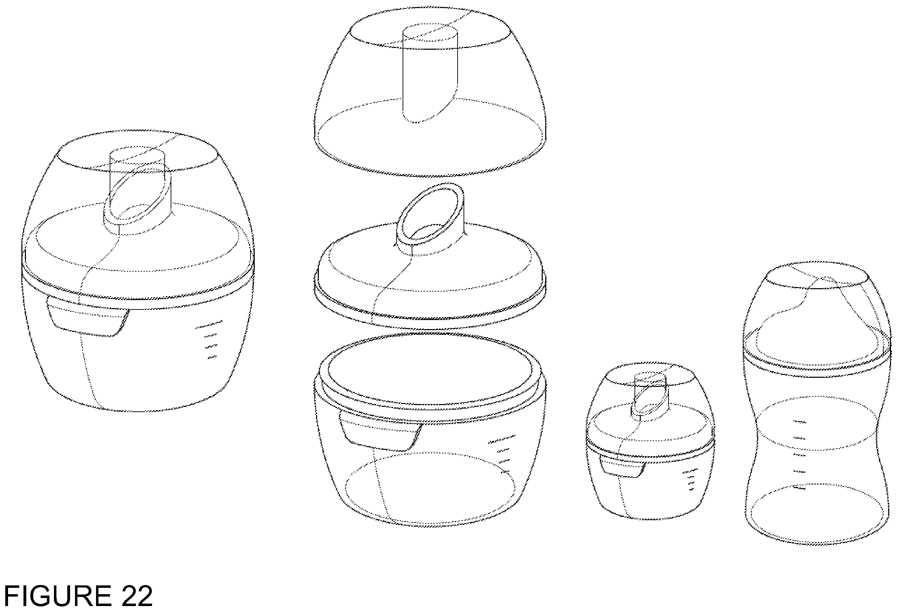

With reference to Figures 21 and 22, the compressible pouch is split into further parts including a milk container 211 that is able to become a separable milk storage solution. A cap, as shown in Figure 22, allows the container to be transported clean and sterile and also allows for secure transport when full with no risk of spills. The container can either be used as a storage device itself or it can be decanted into another bottle.

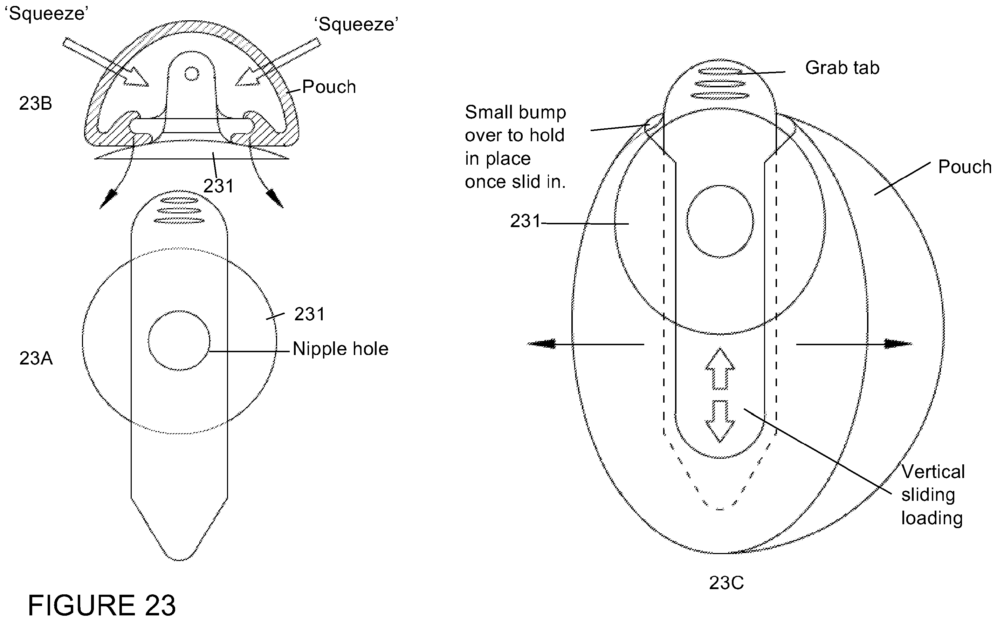

With reference to Figure 23, an optional sealing feature 231 including a nipple hole is provided. The sealing feature 231 is configured to provide a breast interface that seals onto a breast portion around the nipple area. The sealing feature is also configured to attach and seal onto the rear surface of the pouch. The sealing feature 231 is a removable component, which may be squeezed and/or slid along the rear surface of the pouch. The sealing feature 231 provides a breast interface that is removable from the pouch. Hence it is interchangeable allowing customization such as for a different shape or size. A cross sectional view seen from the top of the device 23B is also shown when the sealing feature is inserted in the compressible pouch. The seal provided by the sealing feature is configured not to break when the pouch is squeezed. This is due in part thanks to a gap located between the pouch and the back plate of the sealing feature. A perspective view 23C also illustrates that the sealing feature 231 is able to slid along the rear surface of the pouch. This configuration also allows the compressible pouch to have a large rear opening which is convenient for manufacturing, cleaning and drying.

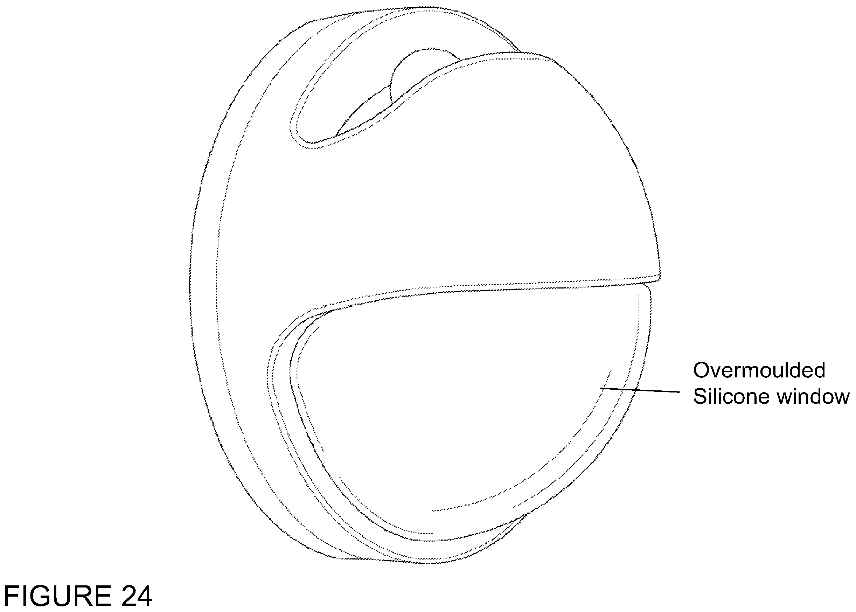

With reference to Figure 24, another passive suction-based milk collection device is provided in which the compressible pouch and frame are integrated as a single part. The silicone compressible pouch and frame are moulded as one single item, using an overmoulding process. In this format, the indicated window is formed from a silicone area overmoulded onto the frame.

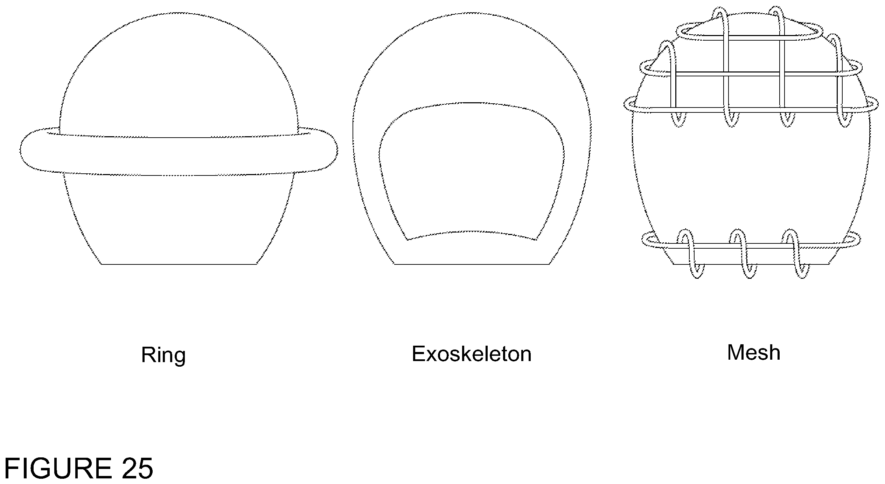

With reference to Figure 25, further variations of the frame structure are provided such as a ring, exoskeleton and mesh structures.

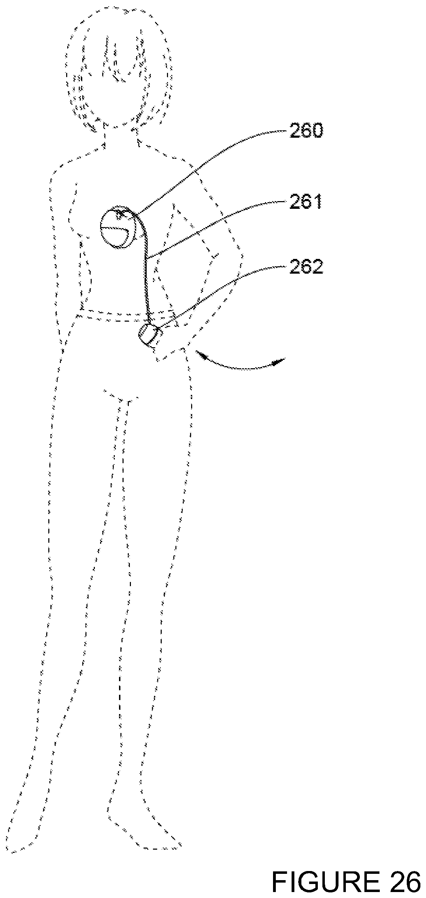

With reference to Figure 26, the silicone breast pump 260 is connected to an external storage device 262 located outside of the user's bra via a tube 261. The system is configured such that any collected milk directly drains from the silicone breast pump to the storage device. The storage device may be for example near the user's waist.

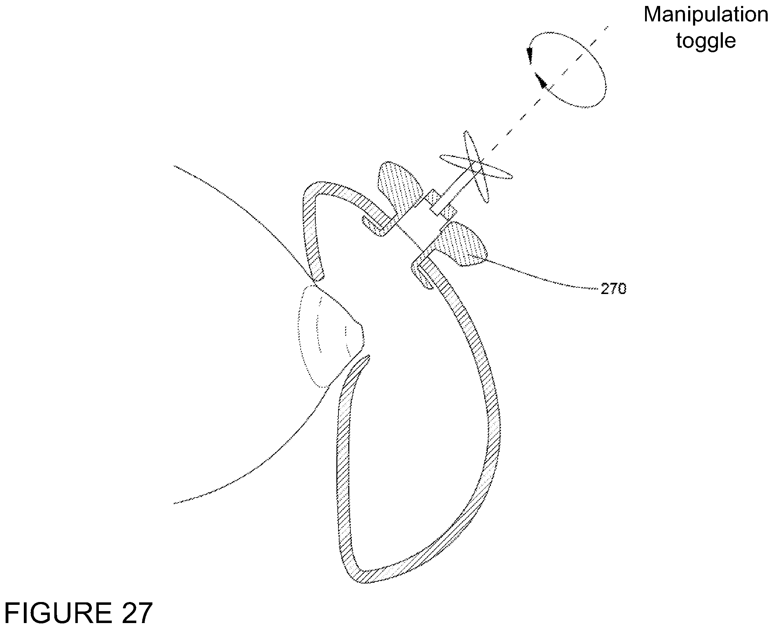

With reference to Figure 27, the compressible pouch includes a valve mechanism and a toggle feature 270. In this configuration, the user interacts with the toggle feature, such as by manually turning or pushing it, in order to open a valve mechanism in a controlled way. A reverse operation of the toggle feature enables the valve to close. Alternatively, it could also be sprung to automatically close.

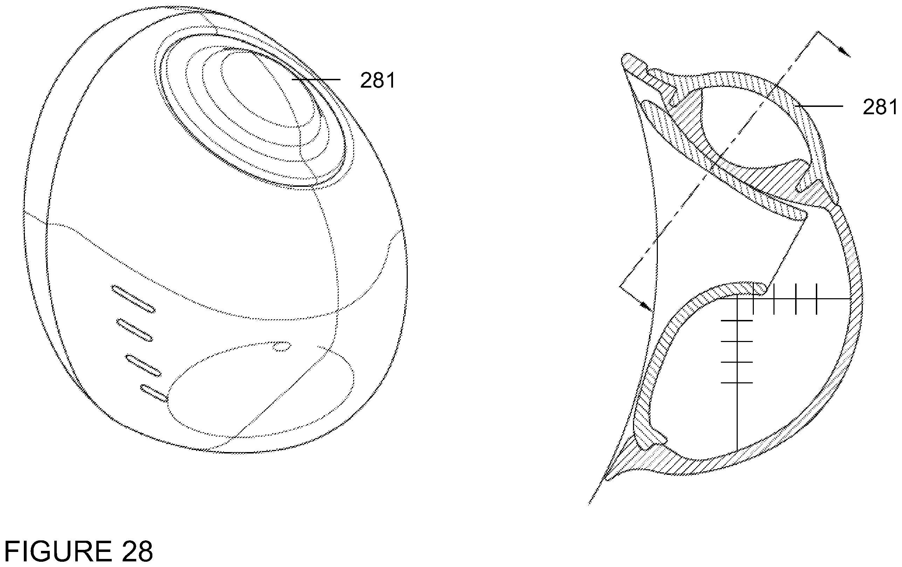

With reference to Figure 28, the passive suction-based milk collection device is further configured to enable a user to increase the suction provided by the device. In this configuration, a vacuum top-up feature 281 is provided at the top of the compressible pouch. The user can manipulate, such as squeeze or push down, the top-up feature in order to top-up the suction of the device.

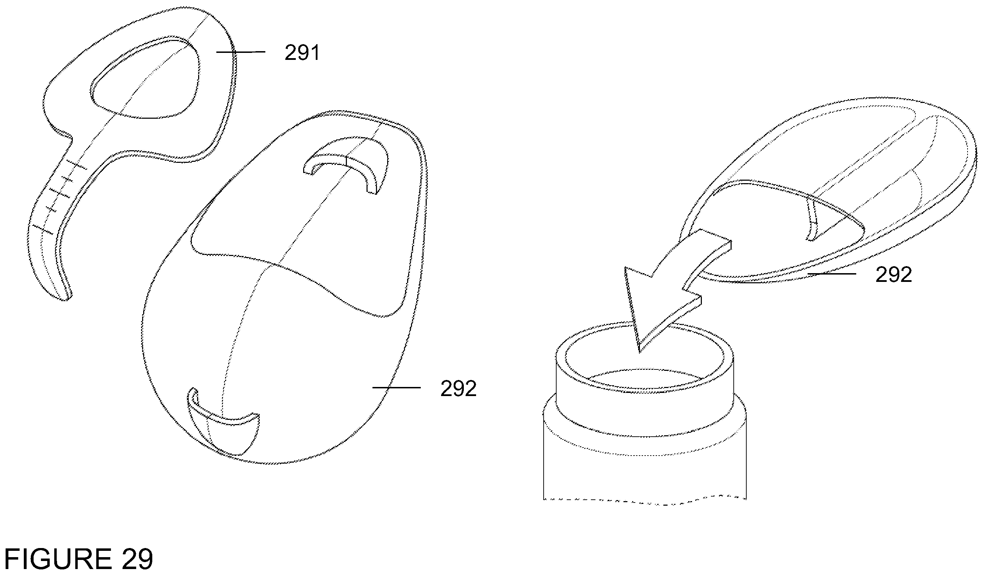

With reference to Figure 29, the compressible pouch includes an external rigid protection 291, which is insertable on or near the outside of pouch. The larger pear-shaped part 292 is a compressible pouch, and the smaller tadpole-shaped part 291 is a rigid portion. The external rigid protection 291 protects against the pressure of a bra. This configuration provides a central rib inside the pouch 292 that offers further structural support for use in-bra. The external rigid protection can optionally be added for greater protection.

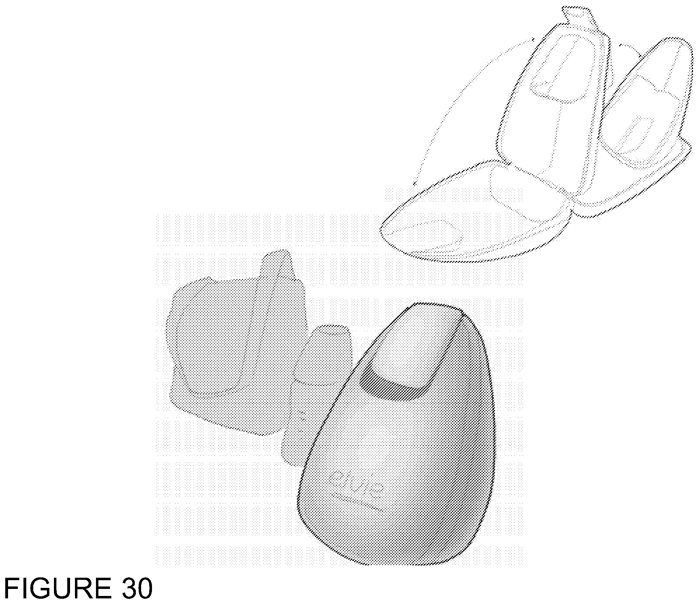

With reference to Figure 30, a carrying case is shown, in which the carrying case is able to transport two passive suction-based milk collection devices at a time, allowing for greater flexibility of storage along with ready-to-go sterilized devices.

### Key features

In this section, we summarise the various features implemented in the passive suction-based collection device. Note that key feature A can be combined with key feature B onwards. All 'Optional' features can be combined with any key features A and B onwards. Each 'Optional' feature may also be an independently novel concept.

### Feature A - In-bra wearable compressible milk pouch with air valve

A wearable passive suction-based milk collection device, the device comprising a compressible milk pouch with a nipple hole or opening in a rear face of the compressible milk pouch, and in which the rear face of the compressible milk pouch is configured to seal against at least part of the user's breast when the pouch is compressed by a user in order to create a negative air pressure inside the pouch, providing suction onto the nipple thereby drawing milk from the nipple for collection in the milk pouch; in which the device is shaped, at least in part, to fit inside a bra. The milk pouch includes or is connected to an air valve to enable the user to reduce the level of negative pressure inside the milk pouch whilst the device is being worn.

Optional features:
- the device includes a rigid or semi-rigid frame or exoskeleton for the compressible milk pouch.

### Feature B - compressible milk pouch with frame

A wearable passive suction-based milk collection device, the device comprising a compressible milk pouch with a nipple hole or opening in a rear face of the compressible milk pouch, and in which the rear face of the compressible milk pouch is configured to seal against at least part of the user's breast when the pouch is compressed by a user in order to create a negative air pressure inside the pouch, providing suction onto the nipple thereby drawing milk from the nipple for collection in the milk pouch;
and the device includes a rigid or semi-rigid frame or exoskeleton configured to retain the compressible milk pouch. The milk pouch includes or is connected to an air valve to enable the user to reduce the level of negative pressure inside the milk pouch whilst the device is being worn.

Optional features:
- the device is shaped, at least in part, to fit inside a bra.

### Feature C - compressible milk pouch with air valve

A wearable passive suction-based milk collection device, the device comprising a compressible milk pouch with a nipple hole or opening in a rear face of the compressible milk pouch, and in which the rear face of the compressible milk pouch is configured to seal against at least part of the user's breast when the pouch is compressed by a user in order to create a negative air pressure inside the pouch, providing suction onto the nipple thereby drawing milk from the nipple for collection in the milk pouch;
and the milk pouch includes or is connected to an air valve configured to enable the user to alter the level of negative pressure inside the milk pouch whilst the device is being worn.

Optional features:
- the device includes a rigid or semi-rigid frame or exoskeleton for the compressible milk pouch.
- the device is shaped, at least in part, to fit inside a bra.

### Optional features for Features A, B and C

### Compressible milk pouch

- compressible milk pouch has dome shaped front face or front face portion
- a bung, stopper or other device is provided to seal the nipple opening in the rear face of the compressible milk pouch
- a front surface of the compressible milk pouch includes a flat portion so that the entire device can rest stably on a flat surface at an angle that prevents milk from spilling from the nipple opening.
- nipple opening in the rear face of the milk pouch also serves as a milk pouring opening.
- compressible milk pouch is a single, shaped piece of silicone.
- compressible milk pouch includes multiple removable and/or interchangeable parts.
- an interchangeable part, including the rear surface of the compressible pouch, enables the user to change the size of the nipple hole.
- compressible milk pouch has a substantially clear or transparent top section, giving an unobstructed view down to the breast for easy nipple alignment.
- compressible milk pouch is substantially clear or transparent
- compressible milk pouch is configured to latch on to the frame
- compressible milk pouch is configured to releasably attach to the frame

### Air valve

- The air valve is removable from the compressible milk pouch.
- The air valve is formed as part of compressible milk pouch.
- Operating the air valve enables air to flow into the compressible milk pouch so as to enable a comfortable removal of the collection device from the breast.
- The air valve is operated via a finger touch.
- A light finger push or squeeze of the non-return valve opens a small cut within the compressible milk pouch, allowing air into the pouch and lowering the vacuum level.
- compressible milk pouch includes or connects to a non-return or one-way air valve
- non-return or one-way air valve allows air to flow into the milk pouch but not air to flow out.
- compressible milk pouch includes or connects to a two-way air valve that both lets air flow into the milk pouch to reduce the negative air pressure and also lets air out of the milk pouch when increasing the negative air pressure.
- an opening in the air valve also serves as a milk pouring opening. The opening in the air valve is opened manually.

### The frame

- frame includes a rear section shaped to fit against a chest or breast
- frame is configured to maintain the rear section of the milk pouch in contact with a breast when the user compresses or deforms the milk pouch to force air out of the milk pouch.
- frame is configured to expose a substantial, exposed surface of the milk pouch when the device is positioned on the breast and that the substantial, exposed surface is squeezed or pressed to create a vacuum to draw milk from the breast and to secure the device in position on the breast
- frame includes a cross bar curving across the front face of the frame
- cross bar divides the front face of the frame into a lower section and an upper section
- the lower section exposes a substantial, exposed surface of the milk pouch when the device is positioned on the breast and that the substantial, exposed surface is squeezed or pressed to create a vacuum to draw milk from the breast and to secure the device in position on the breast
- the upper section exposes the air valve in or connected to the milk pouch
- the upper section provides a view down through the milk pouch and to the nipple.
- The frame is configured to protect the compressible milk pouch from being pushed towards the nipple, such as from being crushed by a bra or any other clothing.
- The frame is configured to provide nipple clearance.
- The frame is configured to enable a higher vacuum level inside the milk pouch, as compared to without using a frame
- The frame is configured to prevent the air valve from accidental activation
- The frame forms an integral part of the compressible milk pouch.
- The frame is entirely separate from the milk pouch
- The frame is entirely external to the milk pouch.
- The frame is at least in part internal to the milk pouch.
- The frame is a semi rigid structure.
- The frame includes a flat base so that the entire device can rest upright on a surface.
- The device includes only two separate pieces; the compressible milk pouch plus the frame for the compressible milk pouch

### Note

It is to be understood that the above-referenced arrangements are only illustrative of the application for the principles of the present invention. Numerous modifications and alternative arrangements can be devised without departing from the scope of the present invention, which is defined in the appended claims.

## Claims

1. A wearable passive suction-based milk collection device (1), the device comprising a compressible milk pouch (10) with a nipple hole or opening (20) in a rear face of the compressible milk pouch, and in which the rear face of the compressible milk pouch is configured to seal against at least part of the user's breast when the pouch is compressed by a user in order to create a negative air pressure inside the pouch, providing suction onto the nipple thereby drawing milk from the nipple for collection in the milk pouch; and in which the device is shaped, at least in part, to fit inside a bra, in which the compressible milk pouch includes or is connected to an air valve (12) to enable the user to reduce the level of negative pressure inside the milk pouch whilst the device is being worn.

2. The device of claim 1, in which the compressible milk pouch has dome shaped front face or front face portion.

3. The device of any preceding claim, in which a front surface of the compressible milk pouch includes a flat portion (13) so that the entire device can rest stably on a flat surface at an angle that prevents milk from spilling from the nipple opening.

4. The device of any preceding claim, in which the nipple opening in the rear face of the milk pouch also serves as a milk pouring opening.

5. The device of any preceding claim, in which the compressible milk pouch is a single, shaped piece of silicone, or includes multiple removable or interchangeable parts, in which one of the interchangeable parts (162), including the rear surface of the compressible pouch, enables the user to change the size of the nipple hole.

6. The device of any preceding claim, in which the compressible milk pouch is substantially clear or transparent or has a substantially clear or transparent top section giving an unobstructed view down to the breast for easy nipple alignment.

7. The device of any preceding claim, in which operating the air valve enables air to flow into the compressible milk pouch so as to decrease the suction on the nipple.

8. The device of any preceding claim, in which the air valve enables air to flow out from the milk pouch when the device is being primed or squeezed and thus reduces air from being substantially evacuated through the nipple opening when the device is being primed or squeezed.

9. The device of any preceding claim, in which the air valve is operated via a finger touch.

10. The device of any of any preceding claim, wherein:
the air valve is a non-return or one-way air valve which allows air to flow into the milk pouch but not air to flow out; or
the air valve is a two-way air valve that both lets air flow into the milk pouch to reduce the negative air pressure and also lets air out of the milk pouch when increasing the negative air pressure.

11. The device of any preceding claim, in which the device includes a rigid or semi-rigid frame or exoskeleton (11) configured to retain the compressible milk pouch.

12. The device of claim 11, in which the compressible milk pouch is configured to releasably attach or latch on to the frame.

13. The device of claims 11 or 12, in which the frame includes a rear section shaped to fit against a chest or breast and/or in which the frame is configured to maintain the rear section of the milk pouch in contact with a breast when the user compresses or deforms the milk pouch to force air out of the milk pouch.

14. The device of any of claims 11 to 13, in which the frame is configured to expose a substantial surface of the milk pouch when the device is positioned on the breast and that the substantial, exposed surface is squeezed or pressed to create a vacuum to draw milk from the breast and to secure the device in position on the breast.

15. The device of any of claims 11 to 14, in which the frame is configured to do one or more of:
protect the compressible milk pouch from being pushed towards the nipple, such as from being crushed by a bra or any other clothing;
provide nipple clearance;
enable a higher vacuum level inside the milk pouch, as compared to without using a frame; and
prevent the air valve from accidental activation.

## Patentansprüche

1. Tragbare passive Absauge-Milchsammelvorrichtung (1), wobei die Vorrichtung einen komprimierbaren Milchbeutel (10) mit einem Brustwarzenloch oder einer Brustwarzenöffnung (20) in einer Rückfläche des komprimierbaren Milchbeutels umfasst und wobei die Rückfläche des komprimierbaren Milchbeutels dazu ausgestaltet ist, gegen mindestens einen Teil der Brust einer Benutzerin abzudichten, wenn der Beutel von einer Benutzerin komprimiert wird, um einen Luftunterdruck in dem Beutel zu erzeugen, so dass die Brustwarze mit Saugung beaufschlagt wird, wodurch Milch aus der Brustwarze zum Sammeln in dem Milchbeutel gesaugt wird, und wobei die Vorrichtung mindestens teilweise so gestaltet ist, dass sie in einen Büstenhalter passt, wobei der komprimierbare Milchbeutel ein Luftventil (12) aufweist oder mit einem Luftventil (12) verbunden ist, damit die Benutzerin den Unterdruckpegel in dem Milchbeutel reduzieren kann, während die Vorrichtung getragen wird.

2. Vorrichtung nach Anspruch 1, wobei der komprimierbare Milchbeutel eine kuppelförmige Vorderfläche oder einen kuppelförmigen Vorderflächenabschnitt hat.

3. Vorrichtung nach einem der vorhergehenden Ansprüche, wobei eine Vorderfläche des komprimierbaren Milchbeutels einen flachen Abschnitt (13) aufweist, so dass die ganze Vorrichtung stabil auf einer flachen Oberfläche in einem Winkel ruhen kann, der verhindert, dass Milch aus der Brustwarzenöffnung herausläuft.

4. Vorrichtung nach einem der vorhergehenden Ansprüche, wobei die Brustwarzenöffnung in der Rückfläche des Milchbeutels auch als eine Milchausgießöffnung dient.

5. Vorrichtung nach einem der vorhergehenden Ansprüche, wobei der komprimierbare Milchbeutel ein einziges geformtes Stück Silikon ist oder mehrere entfernbare oder austauschbare Teile aufweist, wobei die Benutzerin dank eines der austauschbaren Teile (162), einschließlich der Rückfläche des komprimierbaren Beutels, in der Lage ist, die Größe des Brustwarzenlochs zu ändern.

6. Vorrichtung nach einem der vorhergehenden Ansprüche, wobei der komprimierbare Milchbeutel im Wesentlichen klar oder transparent ist oder einen im Wesentlichen klaren oder transparenten oberen Abschnitt hat, durch den man zur leichten Brustwarzenausrichtung ungehindert auf die Brust herabblicken kann.

7. Vorrichtung nach einem der vorhergehenden Ansprüche, wobei dank des Betätigens des Luftventils Luft in den komprimierbaren Milchbeutel strömen kann, um die Saugung an der Brustwarze zu reduzieren.

8. Vorrichtung nach einem der vorhergehenden Ansprüche, wobei dank des Luftventils Luft von dem Milchbeutel ausströmen kann, wenn die Vorrichtung angesaugt oder gequetscht wird, und somit im Wesentlichen weniger Luft durch die Brustwarzenöffnung abgesaugt wird, wenn die Vorrichtung angesaugt oder gequetscht wird.

9. Vorrichtung nach einem der vorhergehenden Ansprüche, wobei das Luftventil durch Berühren mit dem Finger betätigt wird.

10. Vorrichtung nach einem der vorhergehenden Ansprüche, wobei:
das Luftventil ein Rückschlagventil oder Einwegeventil ist, das gestattet, dass Luft in den Milchbeutel strömen, aber keine Luft herausströmen kann, oder
das Luftventil ein Zweiwegeventil ist, das sowohl Luft in den Milchbeutel strömen lässt, um den Luftunterdruck zu reduzieren, als auch Luft aus dem Milchbeutel herauslässt, wenn der Luftunterdruck erhöht wird.

11. Vorrichtung nach einem der vorhergehenden Ansprüche, wobei die Vorrichtung einen starren oder halbstarren Rahmen oder ein starres oder halbstarres Exoskelett (11) aufweist, das bzw. der zum Halten des komprimierbaren Milchbeutels ausgestaltet ist.

12. Vorrichtung nach Anspruch 11, wobei der komprimierbare Milchbeutel zum freigebbaren Anbringen oder Verriegeln an dem Rahmen ausgestaltet ist.

13. Vorrichtung nach Anspruch 11 oder 12, wobei der Rahmen einen hinteren Abschnitt aufweist, der so gestaltet ist, dass er an einen Brustkasten oder eine Brust passt, und/oder wobei der Rahmen dazu ausgestaltet ist, den hinteren Abschnitt des Milchbeutels in Kontakt mit einer Brust zu halten, wenn die Benutzerin den Milchbeutel komprimiert oder deformiert, um Luft aus dem Milchbeutel zu drücken.

14. Vorrichtung nach einem der Ansprüche 11 bis 13, wobei der Rahmen dazu ausgestaltet ist, eine wesentliche Fläche des Milchbeutels freizulegen, wenn die Vorrichtung an der Brust positioniert ist, und dass die wesentliche freiliegende Fläche zur Herstellung eines Vakuums gequetscht oder gedrückt wird, um Milch aus der Brust abzusaugen und die Vorrichtung in Position an der Brust zu befestigen.

15. Vorrichtung nach einem der Ansprüche 11 bis 14, wobei der Rahmen dazu ausgestaltet ist,
den komprimierbaren Milchbeutel davor zu schützen, zu der Brustwarze hin geschoben zu werden, wie etwa davor, dass er durch einen Büstenhalter oder ein anderes Kleidungsstück zusammengedrückt wird,
und/oder
einen Freiraum für die Brustwarze bereitzustellen, und/oder
eine im Vergleich zu einer Nichtverwendung eines Rahmens größere Vakuumhöhe in dem Milchbeutel zu ermöglichen und/oder
eine versehentliche Betätigung des Luftventils zu verhindern.

## Revendications

1. Dispositif portable de collecte de lait par aspiration passive (1), le dispositif comprenant une poche à lait compressible (10) avec un trou ou une ouverture pour le mamelon (20) dans une face arrière de la poche à lait compressible, et dans lequel la face arrière de la poche à lait compressible est configurée pour se sceller contre au moins une partie du sein de l'utilisatrice lorsque la poche est comprimée par une utilisatrice afin de créer une pression d'air négative à l'intérieur de la poche, fournissant une aspiration sur le mamelon, tirant ainsi le lait du mamelon pour le collecter dans la poche à lait ; et dans lequel le dispositif est formé, au moins en partie, pour s'adapter à l'intérieur d'un soutien-gorge, dans lequel la poche à lait compressible comprend ou est reliée à une valve d'air (12) pour permettre à l'utilisatrice de réduire le niveau de pression négative à l'intérieur de la poche à lait pendant que le dispositif est porté.

2. Dispositif selon la revendication 1, dans lequel la poche à lait compressible a une face avant en forme de dôme ou une partie de face avant.

3. Dispositif selon n'importe quelle revendication précédente, dans lequel une surface avant de la poche à lait compressible comprend une partie plate (13) de sorte que l'ensemble du dispositif peut reposer de manière stable sur une surface plate selon un angle qui empêche le lait de s'écouler de l'ouverture du mamelon.

4. Dispositif selon n'importe quelle revendication précédente, dans lequel l'ouverture du mamelon sur la face arrière de la poche à lait sert également d'ouverture de versement du lait.

5. Dispositif selon n'importe quelle revendication précédente, dans lequel la poche à lait compressible est une pièce de silicone unique et façonnée, ou comprend de multiples pièces amovibles ou interchangeables, dans lequel l'une des pièces interchangeables (162), comprenant la face arrière de la poche compressible, permet à l'utilisatrice de modifier la taille du trou de mamelon.

6. Dispositif selon n'importe quelle revendication précédente, dans lequel la poche à lait compressible est sensiblement claire ou transparente ou a une section supérieure sensiblement claire ou transparente donnant une vue dégagée vers le bas jusqu'au sein pour faciliter l'alignement du mamelon.

7. Dispositif selon n'importe quelle revendication précédente, dans lequel l'actionnement de la valve d'air permet à l'air de s'écouler dans la poche à lait compressible de manière à diminuer l'aspiration sur le mamelon.

8. Dispositif selon n'importe quelle revendication précédente, dans lequel la valve d'air permet à l'air de s'écouler de la poche à lait lorsque le dispositif est amorcé ou pressé et réduit ainsi l'air d'être substantiellement évacué par l'ouverture du mamelon lorsque le dispositif est amorcé ou pressé.

9. Dispositif selon n'importe quelle revendication précédente, dans lequel la valve d'air est actionnée par un toucher du doigt.

10. Dispositif selon n'importe quelle revendication précédente,
la valve d'air étant un clapet anti-retour ou à sens unique qui permet à l'air de s'écouler dans la poche à lait, mais pas à l'air de s'en échapper ; ou
la valve d'air étant une valve d'air à deux voies qui laisse entrer l'air dans la poche à lait pour réduire la pression d'air négative et qui laisse également sortir l'air de la poche à lait lors de l'augmentation de la pression d'air négative.

11. Dispositif selon n'importe quelle revendication précédente, dans lequel le dispositif comprend un cadre ou un exosquelette rigide ou semi-rigide (11) configuré pour retenir la poche à lait compressible.

12. Dispositif selon la revendication 11, dans lequel la poche à lait compressible est configurée pour se fixer ou se verrouiller de manière libérable sur le cadre.

13. Dispositif selon les revendications 11 ou 12, dans lequel le cadre comprend une section arrière façonnée pour s'adapter contre une poitrine ou un sein et/ou dans lequel le cadre est configuré pour maintenir la section arrière de la poche à lait en contact avec un sein lorsque l'utilisatrice comprime ou déforme la poche à lait pour forcer l'air à sortir de la poche à lait.

14. Dispositif selon l'une des revendications 11 à 13, dans lequel le cadre est configuré pour exposer une surface substantielle de la poche à lait lorsque le dispositif est positionné sur le sein et que la surface substantielle exposée est pressée ou comprimée pour créer un vide afin d'aspirer le lait du sein et de fixer le dispositif en position sur le sein.

15. Dispositif selon l'une quelconque des revendications 11 à 14, dans lequel le cadre est configuré pour une ou plusieurs actions parmi :
protéger la poche à lait compressible d'une poussée vers le mamelon, par exemple d'un écrasement par un soutien-gorge ou tout autre vêtement ;
permettre un dégagement du mamelon ;
permettre un niveau de vide plus élevé à l'intérieur de la poche à lait, par rapport à l'absence de cadre ; et
empêcher l'activation accidentelle de la valve d'air.
